# EUROPEAN PATENT APPLICATION

(11) **EP 2 415 472 A1**
(43) Date of publication of application: **08.02.2012**
(21) Application number: 10758611.7
(22) Date of filing: 29.03.2010
(51) Int. Cl.: A61K 31/439, A61K 9/19, A61K 47/04, A61K 47/12, A61K 47/18, A61K 47/38, A61P 13/02, A61P 25/00

(54) **SOLID PHARMACEUTICAL COMPOSITION CONTAINING AMORPHOUS BODY OF SOLIFENACIN**

(30) Priority: 30.03.2009 US 202712 P
(71) Applicant: Astellas Pharma Inc., Tokyo 103-8411 (JP)
(72) Inventor: WAKO Yuko, Tokyo 103-8411 (JP); YOSHIHARA Keiichi, Tokyo 103-8411 (JP); ITOU Naoki, Tokyo 103-8411 (JP)
(74) Representative: HOFFMANN EITLE
(86) International application number: PCT/JP2010/055515
(87) International publication number: WO 2010/113840

(57) **Abstract**

The present invention relates to the provision of a stable solid pharmaceutical composition containing an amorphous form of solifenacin or a pharmaceutically acceptable salt thereof and capable of inhibiting decomposition accompanied by long-term storage, to provide the medical field with a formulation of solifenacin or a pharmaceutically acceptable salt thereof. More particularly, the present invention relates to a solid pharmaceutical composition, comprising an amorphous form of solifenacin or a pharmaceutically acceptable salt thereof, and one stabilizer for amorphous solifenacin or two or more stabilizers for amorphous solifenacin, selected from the group consisting of citric acid or a pharmaceutically acceptable salt (excluding a calcium salt) thereof, sodium pyrosulfite, and a pharmaceutically acceptable salt of ethylenediaminetetraacetic acid.

## Description

### TECHNICAL FIELD

The present invention relates to a solid pharmaceutical composition comprising an amorphous form of solifenacin or a pharmaceutically acceptable salt thereof, and a stabilizer for amorphous solifenacin. More particularly, the present invention relates to a solid pharmaceutical composition comprising an amorphous form of solifenacin or a pharmaceutically acceptable salt thereof, and one stabilizer for amorphous solifenacin or two or more stabilizers for amorphous solifenacin, selected from the group consisting of citric acid or a pharmaceutically acceptable salt (excluding a calcium salt) thereof, sodium pyrosulfite, and a pharmaceutically acceptable salt of ethylenediaminetetraacetic acid.
Further, the present invention relates to a process of manufacturing a solid pharmaceutical composition containing an amorphous form of solifenacin or a pharmaceutically acceptable salt thereof.
Furthermore, the present invention relates to use of one stabilizer or two or more stabilizers selected from the group consisting of citric acid or a pharmaceutically acceptable salt (excluding a calcium salt) thereof, sodium pyrosulfite, and a pharmaceutically acceptable salt of ethylenediaminetetraacetic acid, in the manufacture of a stable solid pharmaceutical composition containing an amorphous form of solifenacin or a pharmaceutically acceptable salt thereof.
Furthermore, the present invention relates to a method of stabilizing a solid pharmaceutical composition containing an amorphous form of solifenacin or a pharmaceutically acceptable salt thereof, by adding one stabilizer or two or more stabilizers selected from the group consisting of citric acid or a pharmaceutically acceptable salt (excluding a calcium salt) thereof, sodium pyrosulfite, and a pharmaceutically acceptable salt of ethylenediaminetetraacetic acid to the solid pharmaceutical composition.

### BACKGROUND ART

Solifenacin is represented by the following formula (I): 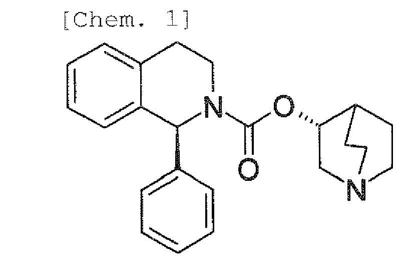 and its chemical name is (R)-quinuclidin-3-yl (S)-1-phenyl-1,2,3,4-tetrahydroisoquinoiine-2-carboxylate.

It has been reported that a series of quinuclidin derivatives, including solifenacin or a pharmaceutically acceptable salt thereof, have an affinity and selectivity against muscarinic M₃ receptors, and are useful as an agent for preventing or treating urinary diseases such as urinary incontinence and pollakiuria in neurogenic pollakiuria, neurogenic bladder, nocturnal enuresis, unstable bladder, cystospasm, and chronic cystitis; respiratory diseases such as chronic obstructive pulmonary diseases, chronic bronchitis, asthma, and rhinitis; and digestive diseases such as irritable bowel syndrome, spastic colitis, and diverticulitis (patent literature 1).
In particular, solifenacin has high selectivity for M₃ receptors located in the smooth muscles, gland tissues, or the like, in comparison with M₂ receptors located in the heart or the like, and is useful as an M₃ receptor antagonist with less side effects on the heart or the like, in particular, as an agent for preventing or treating urinary incontinence and pollakiuria, chronic obstructive pulmonary diseases, chronic bronchitis, asthma, rhinitis, and the like. Solifenacin is placed on the market, as an agent for treating urinary urgency, urinary frequency, and urge incontinence in overactive bladder, as Vesicare (registered trademark) in Japan, VESIcare (registered trademark) in the United States, and Vesicare (registered trademark) in Europe.

Patent literature 1 discloses a process of manufacturing solifenacin hydrochloride (Example 8). Patent literature 1 discloses that the crystal form thereof, which had been crystallized in a mixed solvent of acetonitrile and diethyl ether, had a melting point of 212 to 214°C and an optical rotation of 98.1 ([α]²⁵_{D}, c = 1.00, ethanol).

Patent literature 2 discloses an invention relating to a stable pharmaceutical composition containing solifenacin or a pharmaceutically acceptable salt thereof. This literature discloses a stable pharmaceutical composition containing an amorphous form of solifenacin in an amount less than the specific content, or a pharmaceutical composition of solifenacin or a pharmaceutically acceptable salt thereof containing an inhibitor for amorphization, because the amorphous form of solifenacin tends to degrade by oxidation or the like.

Patent literature 3 discloses a stable granular pharmaceutical composition of solifenacin or a pharmaceutically acceptable salt thereof, which can be obtained by using a binder with a specific glass transition point or melting point, and a granular pharmaceutical composition obtained by carrying out a crystallization promoting treatment after the production of the stable granular pharmaceutical composition.
However, patent literatures 2 and 3 do not disclose techniques capable of stabilizing the amorphous form of solifenacin per se. If the amorphous form can be stabilized, it is useful in providing a pharmaceutical composition containing the amorphous form of solifenacin.

As an invention for stabilizing the amorphous form of solifenacin, patent literature 4 discloses a stable composition containing the amorphous form of solifenacin, and a process of manufacturing the composition.
However, it is known that butylated hydroxytoluene, of which the effect for stabilizing solifenacin was concretely evaluated in the Examples, forms into a quinone structure by NOx or the like and turns to yellow (non-patent literature 1). Further, it is known that a change in color or coloration is easily caused by a metal, in propyl gallate (non-patent literature 2). Therefore, further improvement is necessary to provide a stable pharmaceutical composition containing an amorphous form of solifenacin.

The Ministry of Health, Labor and Welfare in Japan published in June, 2003 the specification of drug products, namely, the concept of degradation products (impurities) in drug products as observed during stability tests (non-patent literature 3). According to the publication, when the amount of the drug substance to be administered per day is less than 10 mg, the threshold of a degradation product requiring safety qualification in a drug product is the lower of either 1.0% as the percentage of the degradation product contained in the drug substance or 50 µg as the total daily intake of the degradation product. When the amount of the drug substance to be administered per day is 10 mg or more to 100 mg or less, the threshold of a degradation product requiring safety qualification in a drug product is the lower of either 0.5% as the percentage of the degradation product contained in the drug substance or 200 µg as the total daily intake of the degradation product. Therefore, when the drug product contains, for example, 5 mg of the drug substance, the specification of a degradation product which can be generally determined without any safety qualification of the degradation product is 1.0% or less as the percentage of the degradation product contained in a drug substance. When the drug product contains, for example, 10 mg of the drug substance, the specification of a degradation product which can be generally determined without any safety qualification of the degradation product is 0.5% or less as the percentage of the degradation product contained in a drug substance.

Solifenacin formulations, which are placed on the market based on the results of clinical trials, are 2.5 mg tables, 5 mg tablets, and 10 mg tablets. To ensure the stability described in non-patent literature 3, the ratio of the main degradation product of solifenacin succinate with respect to the sum of solifenacin succinate and degradation products thereof should be 0.5% by weight or less, in another embodiment, 0.4% by weight or less including variations between product lots or errors caused in the tests.

### CITATION LIST

### PATENT LITERATURE

[patent literature 1] International Publication No. WO96/2019
[patent literature 2] International Publication No. W02005/092889
[patent literature 3] International Publication No. WO2006/070735
[patent literature 4] International Publication No. WO2008/128028

### NON-PATENT LITERATURE

[non-patent literature 1] Polymer Degradation and Stability, 50, 1995, pp. 313-317
[non-patent literature 2] Shokuhin tenka-butsu kotei-syo kaisetsu-syo ("The Commentary of Japanese Standards of Food Additives"), 8th edition, 2007
[non-patent literature 3] Pharmaceutical and Food Safety Bureau, Evaluation and Licensing Division Notification No. 0624001 "Revision of the Guideline on the Impurities in the Medicinal Products with New Active Ingredients"

### SUMMARY OF INVENTION

### TECHNICAL PROBLEM

In formulations containing an amorphous form of solifenacin, solifenacin decomposes time-dependently by the influence of oxidation or the like, and this decomposition is inhibited in the presence of a stabilizer, but discoloration or coloration occurs. An object of the present invention is to provide a stable solid pharmaceutical composition containing an amorphous form of solifenacin or a pharmaceutically acceptable salt thereof, wherein the time-dependent decomposition can be inhibited and no coloration occurs when a formulation of solifenacin or a pharmaceutically acceptable salt thereof is provided for the medical field; and a process of manufacturing the solid pharmaceutical composition.

### SOLUTION TO PROBLEM

To solve the problems, the present inventors converted solifenacin into an amorphous form, and used a water-soluble polymer as a carrier capable of maintaining the amorphous state to prepare an amorphous form of solifenacin. The present inventors physically mixed the amorphous form with propyl gallate as disclosed in patent literature 4, but surprisingly, the expected effect was not observed and the solifenacin decomposed.
Further, in a product prepared by lyophilizing an aqueous solution of solifenacin supplemented with sodium ascorbate, the decomposition of solifenacin proceeded, and the amount of the main degradation product of solifenacin was higher than that in a lyophilized product without sodium ascorbate, and coloration was observed.

In these circumstances, the present inventors paid attention to the stability of an amorphous form of solifenacin, and found that certain compounds could unexpectedly inhibit the time-dependent decomposition of an amorphous form of solifenacin.
Further, the present inventors conducted intensive studies, and found that the specific stabilizers not only can maintain the amorphous state of solifenacin, but also can provide a pharmaceutical composition in which no coloration occurs even after storage under high temperature and high humidity conditions, to complete the present invention.

The present invention provides:
[1] a solid pharmaceutical composition, comprising an amorphous form of solifenacin or a pharmaceutically acceptable salt thereof, and one stabilizer for amorphous solifenacin or two or more stabilizers for amorphous solifenacin, selected from the group consisting of citric acid or a pharmaceutically acceptable salt (excluding a calcium salt) thereof, sodium pyrosulfite, and a pharmaceutically acceptable salt of ethylenediaminetetraacetic acid,
[2] the solid pharmaceutical composition of [1], wherein the amount of the stabilizer for amorphous solifenacin is 0.01% to 500% by weight with respect to the amount of the amorphous form of solifenacin or a pharmaceutically acceptable salt thereof,
[3] the solid pharmaceutical composition of [1], wherein the amount of the stabilizer for amorphous solifenacin is 0.01% to 50% by weight with respect to the total weight of the solid pharmaceutical composition,
[4] the solid pharmaceutical composition of any one of [1] to [3], further comprising a water-soluble polymer,
[5] the solid pharmaceutical composition of [4], wherein the water-soluble polymer has a melting point of 174°C or higher,
[6] the solid pharmaceutical composition of [4] or [5], wherein the water-soluble polymer is a compound or two or more compounds selected from the group consisting of hydroxypropylmethylcellulose, polyvinylpyrrolidone, and methylcellulose,
[7] a process of manufacturing a solid pharmaceutical composition containing an amorphous form of solifenacin or a pharmaceutically acceptable salt thereof, said process comprising the steps of:
   (1) suspending and/or dissolving solifenacin or a pharmaceutically acceptable salt thereof in a pharmaceutically acceptable solvent,
   (2) removing the solvent from the liquid obtained in step (1) to prepare an amorphous form of solifenacin or a pharmaceutically acceptable salt thereof, and
   (3) suspending and/or dissolving one stabilizer for amorphous solifenacin or two or more stabilizers for amorphous solifenacin, selected from the group consisting of citric acid or a pharmaceutically acceptable salt (excluding a calcium salt) thereof, sodium pyrosulfite, and a salt of ethylenediaminetetraacetic acid, in the pharmaceutically acceptable solvent, together with the solifenacin or a pharmaceutically acceptable salt thereof, in step (1); or mixing one stabilizer for amorphous solifenacin or two or more stabilizers for amorphous solifenacin, selected from the group consisting of citric acid or a pharmaceutically acceptable salt (excluding a calcium salt) thereof, sodium pyrosulfite, and a salt of ethylenediaminetetraacetic acid, with the amorphous form of solifenacin or a pharmaceutically acceptable salt thereof obtained in step (2),
[8] use of one stabilizer or two or more stabilizers selected from the group consisting of citric acid or a pharmaceutically acceptable salt (excluding a calcium salt) thereof, sodium pyrosulfite, and a pharmaceutically acceptable salt of ethylenediaminetetraacetic acid, in the manufacture of a stable solid pharmaceutical composition containing an amorphous form of solifenacin or a pharmaceutically acceptable salt thereof, and
[9] a method of stabilizing a solid pharmaceutical composition containing an amorphous form of solifenacin or a pharmaceutically acceptable salt thereof, by adding one stabilizer or two or more stabilizers selected from the group consisting of citric acid or a pharmaceutically acceptable salt (excluding a calcium salt) thereof, sodium pyrosulfite, and a pharmaceutically acceptable salt of ethylenediaminetetraacetic acid to the solid pharmaceutical composition.

### ADVANTAGEOUS EFFECTS OF INVENTION

The present invention shows advantageous effects, for example, that an amorphous form of solifenacin or a pharmaceutically acceptable salt thereof is stable, even after storage under high temperature and high humidity conditions; and that neither discoloration nor coloration occurs in an amorphous form of solifenacin or a pharmaceutically acceptable salt thereof, even after storage under high temperature and high humidity conditions.

### BRIEF DESCRIPTION OF DRAWINGS

[Fig. 1] Figure 1 shows the appearance of the composition prepared in Comparative Example 1 immediately after production.
[Fig. 2] Figure 2 shows the appearances of the compositions prepared in Examples 1 and 2 [Fig. 2(a) and Fig. 2(b), respectively] and Comparative Examples 1 to 3 [Fig. 2(c) to Fig. 2(e), respectively] after storage at 40°C and 75% relative humidity for 1 month.
[Fig. 3] Figure 3 shows a DSC (differential scanning calorimetry) chart of solifenacin succinate salt in a crystalline form.
[Fig. 4] Figure 4 shows a DSC chart of the composition prepared in Comparative Example 1 immediately after production.
[Fig. 5] Figure 5 shows a DSC chart of the composition prepared in Comparative Example 4 immediately after production.
[Fig. 6] Figure 6 shows a DSC chart of the composition prepared in Example 2 after storage at 40°C and 75% relative humidity for 1 month.

### DESCRIPTION OF EMBODIMENTS

Hereinafter embodiments of the present invention will be explained in detail.
The terms "amorphous" and "amorphous form" as used herein with respect to "solifenacin or a pharmaceutically acceptable salt thereof" mean that solifenacin or a pharmaceutically acceptable salt thereof crystallographically has an amorphous structure. The method for evaluating the crystalline state of solifenacin or a pharmaceutically acceptable salt thereof is not particularly limited, so long as the crystalline state can be determined. Examples of the method include a powder X-ray diffraction, a DSC measurement, an NMR measurement, and an infrared spectroscopy measurement. In another embodiment, a DSC measurement may be used. In the case where DSC is used for the analysis, it is defined as amorphous when an endothermic peak at around 147°C derived from a crystalline form of solifenacin is not detected, but it should not be too strictly applied, because measurement conditions can affect the result.
The term "main degradation product" as used herein means the most abundant degradation product in the degradation products from solifenacin. More particularly, the amounts of degradation products contained in a pharmaceutical composition are measured by high performance liquid chromatography, and the product showing the highest measured value in the obtained values is defined as the main degradation product.
The term "coloration" as used herein means color changes into a color other than white or transparency.
The term "under high temperature and high humidity conditions" as used herein means the storage conditions which promote a chemical or physical change in a drug or a formulation. For example, it is defined as the conditions at 40°C and 75% relative humidity.
The term "stable" as used herein means stable against, for example, heat, temperature, and/or humidity. For example, it is defined as a pharmaceutical composition in which, after a solid pharmaceutical composition contained in a high-density polyethylene bottle with a closely sealed metal cap is stored at 40°C and 75% relative humidity for 1 month, the amount of the main degradation product is 0.4% by weight or less with respect to the total weight of the drug.

Hereinafter the composition of the present invention will be explained in detail.
"Solifenacin or a pharmaceutically acceptable salt thereof" or "a salt of solifenacin" used in the present invention means solifenacin or a pharmaceutically acceptable salt thereof. Examples of "a salt of solifenacin" include an acid addition salt with a mineral acid such as hydrochloric acid, hydrobromic acid, hydroiodic acid, sulfuric acid, nitric acid, phosphoric acid, or the like; an acid addition salt with an organic acid such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, ethanesulfonic acid, glutamic acid, or the like; an acid addition salt with an acidic amino acid such as aspartic acid, glutamic acid, or the like; and a quaternary ammonium salt (patent literature 1). In these salts, solifenacin succinate is preferred for providing a medicament.

"Solifenacin or a pharmaceutically acceptable salt thereof" used in the present invention can be easily obtained in accordance with a method disclosed in patent literature 1, a modified method thereof, or a conventional method.

The dose may be individually and appropriately selected in accordance with, for example, an administration route, symptoms, age or sex of the patient. When solifenacin succinate is orally administered to an adult, the daily dose is generally approximately 0.01 mg/kg to 100 mg/kg, which is administered once or divided into two to four doses per day. When it is intravenously administered for certain symptoms, the amount per dose for an adult is generally 0.01 mg/kg to 10 mg/kg, which is administered once or multiple times per day.

The content of solifenacin or a salt thereof may be appropriately selected in accordance with a medical use (indication), and is not particularly limited so long as it is an amount therapeutically or preventively efficient for the medical use. The content per formulation is, for example, 0.05% to 85% by weight, 0.05% to 80% by weight in another embodiment, 0.05% to 50% by weight in still another embodiment, and 0.05% to 10% by weight in another embodiment. The amount of solifenacin contained in the formulation is 0.01 mg to 100 mg, 0.5 mg to 50 mg in another embodiment, and 0.5 mg to 10 mg in still another embodiment.

The stabilizer for "solifenacin or a pharmaceutically acceptable salt thereof" used in the present invention is not particularly limited, so long as it is pharmaceutically acceptable and can stabilize an amorphous form of solifenacin or a pharmaceutically acceptable salt thereof. Examples of the stabilizer include citric acid or a pharmaceutically acceptable salt (excluding a calcium salt) thereof, sodium pyrosulfite, and a pharmaceutically acceptable salt of ethylenediaminetetraacetic acid.

Citric acid or a pharmaceutically acceptable salt (excluding a calcium salt) thereof is not particularly limited, so long as it is a pharmaceutically acceptable salt other than calcium citrate. Examples thereof include citric acid, trisodium citrate, tripotassium citrate, dipotassium hydrogen citrate, isopropyl citrate, and triethyl citrate. In another embodiment, citric acid, trisodium citrate, tripotassium citrate, or triethyl citrate may be used.
The pharmaceutically acceptable salt of ethylenediaminetetraacetic acid is not particularly limited, so long as it is a pharmaceutically acceptable salt other than ethylenediaminetetraacetic acid. Examples thereof include disodium ethylenediaminetetraacetate, calcium disodium ethylenediaminetetraacetate, dipotassium ethylenediaminetetraacetate, tripotassium ethylenediaminetetraacetate, and tetrasodium ethylenediaminetetraacetate. In another embodiment, disodium ethylenediaminetetraacetate, calcium disodium ethylenediaminetetraacetate, or dipotassium ethylenediaminetetraacetate may be used.
These stabilizers for "solifenacin or a pharmaceutically acceptable salt thereof" may be used alone, or as an appropriate combination of two or more thereof.
The content of the stabilizer for "solifenacin or a pharmaceutically acceptable salt thereof" is, for example, 0.01% to 500% by weight, 0.01% to 150% by weight in another embodiment, 0.01% to 100% by weight in still another embodiment, and 0.1% to 50% by weight in still another embodiment, with respect to the weight of an amorphous form of solifenacin or a pharmaceutically acceptable salt thereof. The content of the stabilizer for "solifenacin or a pharmaceutically acceptable salt thereof" contained in the solid pharmaceutical composition is, for example, 0.01% to 50% by weight, 0.01% to 20% by weight in another embodiment, and 0.01% to 10% by weight in still another embodiment.

To maintain the amorphous property of solifenacin or a pharmaceutically acceptable salt thereof in the present invention, a carrier may be further added to the amorphous form. The carrier used is not particularly limited, so long as it can convert solifenacin into an amorphous form, or it can maintain the amorphous form. Examples of the carrier include a water-soluble polymer having a glass transition temperature of 174°C or more, and a water-soluble polymer having a glass transition temperature of 200°C or more in another embodiment. More particularly, examples thereof include hydroxypropylmethylcellulose, polyvinylpyrrolidone, and methylcellulose, and hydroxypropylmethylcellulose in another embodiment. These carriers may be used alone, or as an appropriate combination of two or more thereof.
The content of the carrier is, for example, 0.1 to 20 parts by weight, 0.1 to 10 parts by weight in another embodiment, and 0.1 to 5 parts by weight in still another embodiment, with respect to 1 part by weight of solifenacin or a pharmaceutically acceptable salt thereof.

The solid pharmaceutical composition of the present invention may be formulated by appropriately using one or more various additives if desired. Such additives are not particularly limited, so long as they are pharmaceutically acceptable and pharmacologically acceptable, and include, for example, fillers, binders, disintegrating agents, acidulants, foaming agents, artificial sweeteners, flavors, lubricants, coloring agents, stabilizers, buffers, antioxidants, and surfactants.
Example of the fillers include mannitol, lactose, and crystalline cellulose.
Examples of the binders include an aminoalkyl methacrylate copolymer, ethyl cellulose, hydroxyethylcellulose, hydroxypropylcellulose, hydroxypropylmethylcellulose, polyethylene glycol, polyvinyl alcohol, and polyvinylpyrrolidone.
Examples of the disintegrating agents include corn starch, potato starch, carmellose calcium, carmellose sodium, and low substituted hydroxypropylcellulose.
Examples of the acidulants include tartaric acid and malic acid.
Examples of the foaming agents include sodium bicarbonate.
Examples of the artificial sweeteners include saccharin sodium, dipotassium glycyrrhizinate, aspartame, stevia, and somatin
Examples of the flavors include lemon, lemon lime, orange, and menthol.
Examples of the lubricants include magnesium stearate, calcium stearate, sucrose fatty acid ester, talc, and stearic acid.
Examples of the coloring agents include yellow ferric oxide, red ferric oxide, food yellow No. 4, food yellow No. 5, food red No. 3, food red No. 102, and food blue No. 3.
Examples of the buffers include succinic acid, fumaric acid, tartaric acid, and salts thereof; glutamic acid, glutamine, glycine, aspartic acid, alanine, arginine, and salts thereof; and magnesium oxide, zinc oxide, magnesium hydroxide, phosphoric acid, boric acid, and salts thereof.
Examples of the antioxidants include sodium nitrite, sodium hydrogen sulfite, tocopherol acetate, tocopherol, propyl gallate, ascorbic acid, and sodium ascorbate.
Examples of the surfactants include sodium laurylsulfate, polyoxyethylene sorbitan fatty acid esters (polysorbate 80), and polyoxyethylene hydrogenated castor oil.
These additives may be appropriately added alone, or as a combination of two or more thereof, in an appropriate amount(s).

In the present invention, any conventional process, apparatus, and means may be appropriately selected and are not particularly limited. The formulation form is not particularly limited, so long as it is a solid pharmaceutical composition, and various forms such as particles, granules, tablets, and a lyophilized product may be selected.

In the case that the obtained formulation is a tablet, the tablet may be subjected to a coating treatment, if desired. As a coating base, for example, hydroxypropylmethylcellulose, talc, titanium dioxide, yellow ferric oxide, or lactose may be appropriately added alone, or as a combination of two or more thereof, in an appropriate amount(s). As a method for coating, a film-coating may be carried out, for example, by dissolving or suspending the coating base in a solvent such as water and spraying the tablet with the solution or suspension in a pan coating apparatus.

Hereinafter, typical processes for manufacturing the solid pharmaceutical composition of the present invention will be explained.
(1) Step of suspending and/or dissolving solifenacin or a pharmaceutically acceptable salt thereof
   Solifenacin or a pharmaceutically acceptable salt thereof is suspended and/or dissolved in a pharmaceutically acceptable solvent. The solvent is not particularly limited, so long as solifenacin can be dissolved. Examples of the solvent include water and/or an organic solvent, and include water, methanol, ethanol, and acetone in another embodiment. These solvents may be used alone, or as a mixture of two or more solvents. In addition to solifenacin or a pharmaceutically acceptable salt thereof, a carrier and/or a stabilizer for an amorphous form of solifenacin may be added to the solution or the suspension.
(2) Step of obtaining an amorphous form of solifenacin or a pharmaceutically acceptable salt thereof by evaporating the solvent
   This step is not particularly limited, so long as an amorphous form of solifenacin or a pharmaceutically acceptable salt thereof may be prepared by evaporating the solvent from the liquid prepared in step (1). Examples of a method which may be used in this step include lyophilization and spray drying. In lyophilization, the solution or the suspension is allowed to stand in a freezer or the like to be frozen, and then, the solvent is removed by suction under vacuum. In spray drying, the solvent contained in the solution or the suspension is removed using a spray dryer. After this step, a step of adding a stabilizer for an amorphous form of solifenacin to solifenacin or a pharmaceutically acceptable salt thereof, in which the solvent is removed, may be further carried out.
   In addition, if desired, the solid pharmaceutical composition of the present invention may be mixed with one or more additives and the resulting mixture may be compression-molded, or an amorphous form of solifenacin or a pharmaceutically acceptable salt thereof may be mixed with one or more appropriate additives and the resulting mixture may be granulated. Examples of a method of preparing granulated products include a high-shear granulation method, a fluidized bed granulation method, a tumbling granulation method, and a dry granulation method.

### EXAMPLES

The present invention will be further illustrated by, but is by no means limited to, the following Examples and Comparative Examples.

### (Example 1)

A drug solution was prepared by dissolving 10 parts of solifenacin succinate, 0.2 parts of citric acid (KANTO CHEMICAL CO., INC.), and 10 parts of hydroxypropylmethylcellulose (TC-5E, Shin-Etsu Chemical Co., Ltd.) in 250 parts of water while stirring. The resulting solution was frozen in a freezer at -80°C for 2 hours and then dehydrated under vacuum using a freeze dryer (FD-81 EYELA, Tokyo Rikakikai Co., LTD.) for 48 hours to obtain a pharmaceutical composition of the present invention.

### (Example 2)

A drug solution was prepared by dissolving 10 parts of solifenacin succinate, 10 parts of ethylenediaminetetraacetic acid disodium salt (KANTO CHEMICAL CO., INC.), and 3 parts of hydroxypropylmethylcellulose (TC-5E, Shin-Etsu Chemical Co., Ltd.) in 246 parts of water while stirring. The resulting solution was frozen in a freezer at -80°C for 2 hours and then dehydrated under vacuum using a freeze dryer (FD-81 EYELA, Tokyo Rikakikai Co., LTD.) for 48 hours to obtain a pharmaceutical composition of the present invention.

### (Example 3)

A drug solution was prepared by dissolving 10 parts of solifenacin succinate, 10 parts of citric acid (KANTO CHEMICAL CO., INC.), and 3 parts of hydroxypropylmethylcellulose (TC-5E, Shin-Etsu Chemical Co., Ltd.) in 246 parts of water while stirring. The resulting solution was frozen in a freezer at -80°C for 2 hours and then dehydrated under vacuum using a freeze dryer (FD-81 EYELA, Tokyo Rikakikai Co., LTD.) for 48 hours to obtain a pharmaceutical composition of the present invention.

### (Example 4)

drug solution was prepared by dissolving 10 parts of solifenacin succinate, 10 parts of tripotassium citrate (KANTO CHEMICAL CO., INC.), and 3 parts of hydroxypropylmethylcellulose (TC-5E, Shin-Etsu Chemical Co., Ltd.) in 246 parts of water while stirring. The resulting so7.ution was frozen in a freezer at -80°C for 2 hours and then dehydrated under vacuum using a freeze dryer (FD-81 EYELA, Tokyo Rikakikai Co., LTD.) for 48 hours to obtain a pharmaceutical composition of the present invention.

### (Example 5)

A drug solution was prepared by dissolving 10 parts of solifenacin succinate, 10 parts of triethyl citrate (CANTO CHEMICAL CO., INC.), and 3 parts of hydroxypropylmethylcellulose (TC-5E, Shin-Etsu Chemical Co., Ltd.) in 246 parts of water while stirring. The resulting solution was frozen in a freezer at -80°C for 2 hours and then dehydrated under vacuum using a freeze dryer (FD-81 EYELA, Tokyo Rikakikai Co., LTD.) for 48 hours to obtain a pharmaceutical composition of the present invention.

### (Example 6)

A drug solution was prepared by dissolving 10 parts of solifenacin succinate, 10 parts of trisodium citrate (KANTO CHEMICAL CO., INC.), and 3 parts of hydroxypropylmethylcellulose (TC-5E, Shin-Etsu Chemical Co., Ltd.) in 246 parts of water while stirring. The resulting solution was frozen in a freezer at -80°C for 2 hours and then dehydrated under vacuum using a freeze dryer (FD-81 EYELA, Tokyo Rikakikai Co., LTD.) for 48 hours to obtain a pharmaceutical composition of the present invention.

### (Example 7)

A drug solution was prepared by dissolving 10 parts of solifenacin succinate, 10 parts of
ethylenediaminetetraacetic acid calcium disodium salt (KANTO CHEMICAL CO., INC.), and 3 parts of
hydroxypropylmethylcellulose (TC-5E, Shin-Etsu Chemical Co., Ltd.) in 246 parts of water while stirring. The resulting solution was frozen in a freezer at -80°C for 2 hours and then dehydrated under vacuum using a freeze dryer (FD-81 EYELA, Tokyo Rikakikai Co., LTD.) for 48 hours to obtain a pharmaceutical composition of the present invention.

### (Example 8)

A drug solution was prepared by dissolving 10 parts of solifenacin succinate, 10 parts of
ethylenediaminetetraacetic acid dipotassium salt (KANTO CHEMICAL CO., INC.), and 3 parts of
hydroxypropylmethylcellulose (TC-5E, Shin-Etsu Chemical Co., Ltd.) in 246 parts of water while stirring. The resulting solution was frozen in a freezer at -80°C for 2 hours and then dehydrated under vacuum using a freeze dryer (FD-81 EYELA, Tokyo Rikakikai Co., LTD.) for 48 hours to obtain a pharmaceutical composition of the present invention.

### (Example 9)

A drug solution was prepared by dissolving 10 parts of solifenacin succinate and 10 parts of
hydroxypropylmethylcellulose (TC-5E, Shin-Etsu Chemical Co., Ltd.) in 200 parts of water while stirring. The resulting solution was sprayed using a spray dryer to obtain drug particles. The drug particles were mixed with 10 parts of sodium pyrosulfite to obtain a pharmaceutical composition of the present invention.

### (Comparative Example 1)

A drug solution was prepared by dissolving 10 parts of solifenacin succinate and 3 parts of
hydroxypropylmethylcellulose (TC-5E, Shin-Etsu Chemical Co., Ltd.) in 246 parts of water while stirring. The resulting solution was frozen in a freezer at -80°C for 2 hours and then dehydrated under vacuum using a freeze dryer (FD-81 EYELA, Tokyo Rikakikai Co., LTD.) for 48 hours to obtain a pharmaceutical composition for comparison.

### (Comparative Example 2)

A drug solution was prepared by dissolving 10 parts of solifenacin succinate, 10 parts of sodium isoascorbate (KANTO CHEMICAL CO., INC.), and 3 parts of
hydroxypropylmethylcellulose (TC-5E, Shin-Etsu Chemical Co., Ltd.) in 246 parts of water while stirring. The resulting solution was frozen in a freezer at -80°C for 2 hours and then dehydrated under vacuum using a freeze dryer (FD-81 EYELA, Tokyo Rikakikai Co., LTD.) for 48 hours to obtain a pharmaceutical composition for comparison.

### (Comparative Example 3)

A drug solution was prepared by dissolving 10 parts of solifenacin succinate, 10 parts of sodium ascorbate (KANTO CHEMICAL CO., INC.), and 3 parts of
hydroxypropylmethylcellulose (TC-5E, Shin-Etsu Chemical Co., Ltd.) in 246 parts of water while stirring. The resulting solution was frozen in a freezer at -80°C for 2 hours and then dehydrated under vacuum using a freeze dryer (FD-81 EYELA, Tokyo Rikakikai Co., LTD.) for 48 hours to obtain a pharmaceutical composition for comparison.

### (Comparative Example 4)

A drug solution was prepared by dissolving 10 parts of solifenacin succinate and 10 parts of
hydroxypropylmethylcellulose (TC-5E, Shin-Etsu Chemical Co., Ltd.) in 200 parts of water while stirring. The resulting solution was sprayed using a spray dryer to obtain a pharmaceutical composition for comparison.

### (Comparative Example 5)

A drug solution was prepared by dissolving 10 parts of solifenacin succinate and 10 parts of
hydroxypropylmethylcellulose (TC-5E, Shin-Etsu Chemical Co., Ltd.) in 200 parts of water while stirring. The resulting solution was sprayed using a spray dryer to obtain drug particles. The drug particles were mixed with 10 parts of calcium citrate to obtain to obtain a pharmaceutical composition for comparison.

### (Comparative Example 6)

A drug solution was prepared by dissolving 10 parts of solifenacin succinate and 10 parts of
hydroxypropylmethylcellulose (TC-5E, Shin-Etsu Chemical Co., Ltd.) in 200 parts of water while stirring. The resulting solution was sprayed using a spray dryer to obtain drug particles. The drug particles were mixed with 10 parts of propyl gallate to obtain to obtain a pharmaceutical composition for comparison.

### (Comparative Example 7)

A drug solution was prepared by dissolving 10 parts of solifenacin succinate, 10 parts of
ethylenediaminetetraacetic acid (KANTO CHEMICAL CO., INC.), and 3 parts of hydroxypropylmethylcellulose (TC-5E, Shin-Etsu Chemical Co., Ltd.) in 246 parts of water while stirring. The resulting solution was frozen in a freezer at -80°C for 2 hours and then dehydrated under vacuum using a freeze dryer (FD-81 EYELA, Tokyo Rikakikai Co., LTD.) for 48 hours to obtain a pharmaceutical composition for comparison.

### (Experimental Example 1) Stability test

The compositions prepared in Examples 1 to 9 and Comparative Examples 1 to 7 were put into high-density polyethylene bottles with a metal cap, and each bottle was closely sealed with the metal cap and stored at 40°C and 75% relative humidity for 1 month. After the storage, the amount of the main degradation product contained in each bottle was measured. The results were shown in Table 1. The amounts of degradation products generated during the storage were measured by high performance liquid chromatography, and a degradation product with the highest value was regarded as the main degradation product.

As shown in Table 1, 3.00% by weight of the main degradation product was detected after only 1 month from the beginning of the storage when no stabilizer was added (Comparative Example 1), but a smaller amount (0.4% by weight or less) of the main degradation product was detected in all of the Examples in comparison with Comparative Example 1. The main degradation products detected in all of the Comparative Examples exceeded 2.5%, and the inhibitory effect on decomposition was not observed. In particular, the amount of the main degradation product was 4.60% by weight and 3.22% by weight when sodium ascorbate (Comparative Example 3) and ethylenediaminetetraacetic acid (Comparative Example 7) were added, respectively, and an increase in the main decomposition products was unexpectedly detected in comparison with no addition (Comparative Example 1).

**[Table 1]**

| Results in stability test of compositions containing amorphous solifenacin succinate | | |
|---|---|---|
| | Amount of main degradation product (% by weight) | |
| | Before storage | 1 Month |
| Example 1 | **ND** | 0. 15 |
| Example 2 | **ND** | 0. 02 |
| Example 3 | **ND** | **ND** |
| Example 4 | 0. 04 | 0. 16 |
| Example 5 | **ND** | 0. 25 |
| Example 6 | 0. 03 | 0. 25 |
| Example 7 | **ND** | 0. 22 |
| Example 8 | 0. 02 | 0. 03 |
| Example 9 | **ND** | **ND** |
| Comparative Example 1 | **ND** | 3. 00 |
| Comparative Example 2 | 0.03 | 2. 84 |
| Comparative Example 3 | 0. 03 | 4. 60 |
| Comparative Example 4 | **ND** | 7. 61 |
| Comparative Example 5 | **ND** | 6. 64 |
| Comparative Example 6 | **ND** | 2. 59 |
| Comparative Example 7 | **ND** | 3. 22 |

| | | |
|---|---|---|
| **ND**: not detected (<0.02%) | | |

### (Experimental Example 2) Evaluation of coloring

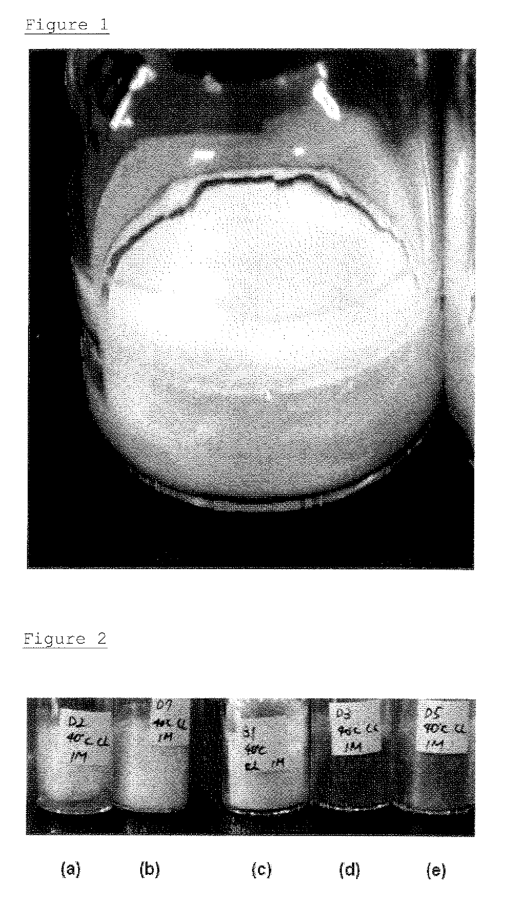

The appearance of the composition prepared in Comparative Example 1 at the beginning of storage is shown in Figure 1.
With respect to the compositions described in Examples 1 and 2 and Comparative Examples 1 to 3, these compositions were prepared in glass vials with a plastic cap, and put into high-density polyethylene bottles with a metal cap, and each bottle was closely sealed with the metal cap and stored at 40°C and 75% relative humidity for 1 month. After storage, the appearances thereof were observed. The results are shown in Figure 2.

As shown in Figure 1, the composition was white at the beginning of storage. In the test at 40°C and 75% relative humidity, the compositions described in Comparative Example 2 [Figure 2, (d)] and Comparative Example 3 [Figure 2, (e)] changed to brown after only 1 month of storage. By contrast, the compositions described in Examples 1 to 3 [Figure 2, (a), (b), and (c)] did not change color, and had the same appearance as immediately after the preparation.

### (Experimental Example 3) Evaluation of crystalline properties

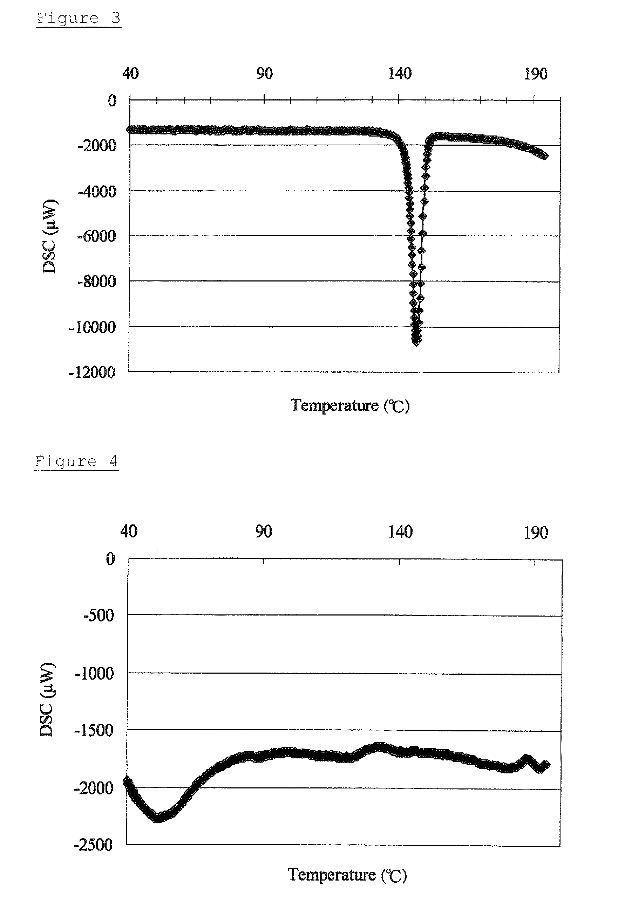

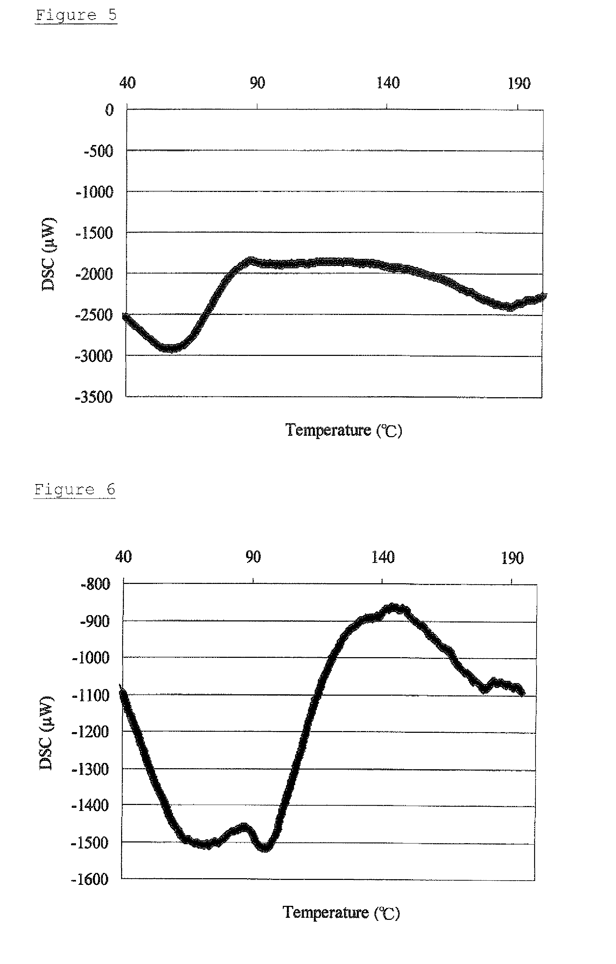

With respect to solifenacin succinate salt in a crystalline form (Figure 3), the compositions of Comparative Examples 1 and 4 at the beginning of storage (Figures 4 and 5, respectively), the composition of Example 2 after storage (Figure 6), DSC was used to evaluate their crystalline properties. As shown in Figure 3, it was found that the crystalline form of solifenacin succinate salt had an endothermic peak at around 147°C. Further, it was found from Figures 4 and 5 that the amorphous form of solifenacin succinate salt did not have any endothermic peaks. Furthermore, it was confirmed from Figure 6 that the composition of Example 2 was in the amorphous form, because the endothermic peak at around 147°C was not observed after storage for 1 month.

### INDUSTRIAL APPLICABILITY

A technical feature of the present invention resides in the industrially remarkable effect that a solid pharmaceutical composition which is stable for long-term storage, containing solifenacin or a pharmaceutically acceptable salt thereof, can be provided by using a specific stabilizer. Further, the present invention is useful as a technique capable of providing various stable formulations containing solifenacin or a pharmaceutically acceptable salt thereof, the development of which is desired as a potent medicament for urinary incontinence and pollakiuria, by using the pharmaceutical composition of the present invention.
As stated above, the present invention was explained with reference to particular embodiments, but modifications and improvements obvious to those skilled in the art are included in the scope of the present invention.

## Claims

1. A solid pharmaceutical composition, comprising an amorphous form of solifenacin or a pharmaceutically acceptable salt thereof, and one stabilizer for amorphous solifenacin or two or more stabilizers for amorphous solifenacin, selected from the group consisting of citric acid or a pharmaceutically acceptable salt (excluding a calcium salt) thereof, sodium pyrosulfite, and a pharmaceutically acceptable salt of ethylenediaminetetraacetic acid.

2. The solid pharmaceutical composition according to claim 1, wherein the amount of the stabilizer for amorphous solifenacin is 0.01% to 500% by weight with respect to the amount of the amorphous form of solifenacin or a pharmaceutically acceptable salt thereof.

3. The solid pharmaceutical composition according to claim 1, wherein the amount of the stabilizer for amorphous solifenacin is 0.01% to 50% by weight with respect to the total weight of the solid pharmaceutical composition.

4. The solid pharmaceutical composition according to any one of claims 1 to 3, further comprising a water-soluble polymer.

5. The solid pharmaceutical composition according to claim 4, wherein the water-soluble polymer has a melting point of 174°C or higher.

6. The solid pharmaceutical composition according to claim 4 or 5, wherein the water-soluble polymer is a compound or two or more compounds selected from the group consisting of hydroxypropylmethylcellulose, polyvinylpyrrolidone, and methylcellulose.

7. A process of manufacturing a solid pharmaceutical composition containing an amorphous form of solifenacin or a pharmaceutically acceptable salt thereof, said process comprising the steps of:
(1) suspending and/or dissolving solifenacin or a pharmaceutically acceptable salt thereof in a pharmaceutically acceptable solvent,
(2) removing the solvent from the liquid obtained in step (1) to prepare an amorphous form of solifenacin or a pharmaceutically acceptable salt thereof, and
(3) suspending and/or dissolving one stabilizer for amorphous solifenacin or two or more stabilizers for amorphous solifenacin, selected from the group consisting of citric acid or a pharmaceutically acceptable salt (excluding a calcium salt) thereof, sodium pyrosulfite, and a salt of ethylenediaminetetraacetic acid, in the pharmaceutically acceptable solvent, together with the solifenacin or a pharmaceutically acceptable salt thereof, in step (1); or mixing one stabilizer for amorphous solifenacin or two or more stabilizers for amorphous solifenacin, selected from the group consisting of citric acid or a pharmaceutically acceptable salt (excluding a calcium salt) thereof, sodium pyrosulfite, and a salt of ethylenediaminetetraacetic acid, with the amorphous form of solifenacin or a pharmaceutically acceptable salt thereof obtained in step (2).

8. Use of one stabilizer or two or more stabilizers selected from the group consisting of citric acid or a pharmaceutically acceptable salt (excluding a calcium salt) thereof, sodium pyrosulfite, and a pharmaceutically acceptable salt of ethylenediaminetetraacetic acid, in the manufacture of a stable solid pharmaceutical composition containing an amorphous form of solifenacin or a pharmaceutically acceptable salt thereof.

9. A method of stabilizing a solid pharmaceutical composition containing an amorphous form of solifenacin or a pharmaceutically acceptable salt thereof, by adding one stabilizer or two or more stabilizers selected from the group consisting of citric acid or a pharmaceutically acceptable salt (excluding a calcium salt) thereof, sodium pyrosulfite, and a pharmaceutically acceptable salt of ethylenediaminetetraacetic acid to the solid pharmaceutical composition.
